## Europäisches Patentamt

(19) **European Patent Office**

**Office européen des brevets**

(11) Publication number: **0 198 173**
**B1**

(12) **EUROPEAN PATENT SPECIFICATION**

(45) Date of publication of patent specification: **09.05.90**

(51) Int. Cl.⁵: **C 08 L 23/12, A 61 L 2/08**

(21) Application number: **86102076.6**

(22) Date of filing: **18.02.86**

(54) Prevention of odor generation during gamma-irradiation of polypropylen fibres.

(30) Priority: **13.03.85 US 711414**

(43) Date of publication of application:
**22.10.86 Bulletin 86/43**

(45) Publication of the grant of the patent:
**09.05.90 Bulletin 90/19**

(84) Designated Contracting States:
**AT BE CH DE FR GB IT LI NL SE**

(56) References cited:
**US-A-3 313 754**
**US-A-3 598 776**
**US-A-3 695 917**
**US-A-3 758 273**

(73) Proprietor: **HERCULES INCORPORATED**
**Hercules Plaza**
**Wilmington Delaware 19894 (US)**

(72) Inventor: **Burch, George Nelson Blair**
**P.O. Box 1102**
**Covington Georgia 30209 (US)**

(74) Representative: **Wey, Hans-Heinrich, Dipl.-Ing.**
**et al**
**Patentanwälte Wey & Partner**
**Widenmayerstrasse 49**
**D-8000 München 22 (DE)**

Courier Press, Leamington Spa, England.

# EP 0 198 173 B1

**Description**

This invention relates to stabilized polypropylene compositions useful in preparing shaped articles having a reduced tendency toward the accumulation of malodorous decomposition products during and following exposure to gamma-radiation from a cobalt 60 source.

Cobalt 60 gamma-irradiation has become an accepted sterilization technique for medical devices. A dose rate of 2.5 Mrads is customary, although dosages as high as $5.5 \cdot 10^4$ Gy may be used. For shaped polymeric articles such as syringes, tubing, tissue culture flasks, packaging film, etc., degradation during or subsequent to irradiation results in problems such as embrittlement, discoloration and reduced heat and light stability.

U.S. Patents 4,110,185, 4,274,932 and 4,467,065 disclose stabilization of polymers against embrittlement during or subsequent to irradiation by incorporating a mobilizer such as a hydrocarbon oil, halogenated hydrocarbon oil, phthalic ester oil, vegetable oil, or silicone oil.

European patent application 78,603 discloses the use of a hindered amine or its salt, N-oxide, N-hydroxide or N-nitroxide to stabilize polyolefin medical articles, e.g., syringes, against yellowing and/or embrittlement resulting from radiation sterilization.

British Patent 1,050,802 discloses the use of a stabilizer system comprising an organic carboxylic acid, e.g., adipic or benzoic acid, and an organic phosphite for articles such as disposable syringes made from radiation sterilizable polyolefin compositions.

U.S. 4,460,445 discloses an olefinic polymer composition containing a hindered phenolic stabilizer and a benzaldehyde acetal which is resistant to degradation when subjected to sterilizing amounts of radiation.

Polypropylene compositions containing 1% of a rosin ester as a stabilizer are already known from US—A—3 313 754.

It is known to use polypropylene fibers for medical applications such as gauze, bottle stuffing, a component of nonwoven products such as surgical gowns and operating room barriers, and in combination with other fibers in fabrics. These products, packaged in plastic films, paper envelopes or glass vials, have been sterilized by irradiation, a method which is both economical and convenient. However, a major obstacle to commercial acceptance of these fibrous products is the disagreeable odor generated upon irradiation. Odor may also be a problem in polypropylene shaped articles other than fibers, e.g. molded articles, film for packaging etc. None of the prior art stabilizer systems addresses the problem of odor generation.

According to the invention, a polypropylene composition useful in preparing shaped articles having a reduced tendency toward odor generation upon sterilization of the article by gamma-irradiation, and comprising stabilizing material in a major amount of polypropylene, is characterized in that it comprises from 0.095 to 0,50% by weight of a rosin ester as a stabilizer.

Other stabilizers normally used to prevent degradation of polyolefins may also be present.

Analysis of irradiated polypropylene fibers suggests that the liberated odor-producing entities may be oxidation fragments having a low molecular weight and high vapor pressure, e.g., formic, acetic or butyric acids, generated by scission of the polymer chain.

The stabilizers for use in the polypropylene compositions of this invention are rosin esters inclusive of lower alkyl esters such as methyl, ethyl and propyl, and are characterized by the presence of nonconjugated double bonds within their cyclic structures. Although we do not intend to limit the present invention by theoretical considerations, it is believed that these stabilizers may be preferentially attacked by ozone and/or oxygen during irradiation, thereby reducing the amount of malodorous low molecular weight products split off from the polymer chain. The stabilizers of this invention are effective in maintaining good physical properties in the finished product as well as providing odor control.

The rosin from which the odor-suppressing stablizers of this invention are derived is generally defined on page 586 of Hackh's Chemical Dictionary, 4th edition, and is inclusive of any of the commercially available types of rosin such as wood rosin, gum rosin, tall oil rosin and mixtures thereof in their crude or refined state. The term "rosin" as used here also includes modified rosins such as partially hydrogenated rosin and partially disproportionated rosin.

The rosin esters useful as the odor-suppressing stabilizers of this invention are preferably esters of a monohydric alcohol and rosin acids such as abietic acid. The methyl ester of rosin and the methyl ester of hydrogenated rosin are most preferred.

The odor suppressing stabilizers are present in the composition of this invention in amounts of from 0.095 to 0.50% by weight.

In addition to the odor-suppressing stabilizer, other stabilisers commonly used to prevent degradation of polyolefins may also be present. These may include an antioxidant, a prodegradant to reduce the molecular weight of the polymer and improve processability, a light stabilizer, and/or an antacid to protect processing equipment. Examples of antioxidants, prodegradants, light stabilizers and antacids suitable for use with polyolefins are well known to those skilled in the art. While such other additives may be helpful, they are not essential for the purpose of this invention.

The polypropylene is preferably used in flake form, i.e., a powder resembling flour, to facilitate dispersion of the odor-suppressing stabilizer, and any other additives that may be used, in the polymer. Additives do not mix well with other commercially available forms of polypropylene, e.g., polypropylene

2

chips. Preferably polypropylene is mixed with such additives in an impact blender and the mixture is then extruded and shaped, e.g., by spinning.

The polypropylene used in the composition of this invention is characterized by a crystallinity of from 55% to 65%, a weight average molecular weight $M_w$ of from $3.0 \times 10^5$ to $4.0 \times 10^5$, a molecular weight distribution of from 5.0 to 8.0 and a melt flow of from 2.5 to 4.0 g/10 minutes at 230°C.

All percentages in the following examples are % by weight, based on the total weight of the composition.

### Example 1

96.23% Polypropylene in flake form (crystallinity 60%, $M_w$ $3.5 \times 10^5$, molecular weight distribution 6.4, melt flow 3.2 g/10 min) is mixed in an impact blender with 0.06% 1,3,5-tris(4-tert butyl-3-hydroxy-2,6-dimethylbenzyl)-1,3,5-triazine-2,4,6-(1H,3H,5H)-trione as an antioxidant, 0.10% calcium stearate as an antacid, 0.034% 2,5-dimethyl-2,5-di(t-butylperoxy) hexane as a prodegradant, 0.097% poly-[6-(1,1,3,3-tetramethylbutyl)-amino]-1,3,5-triazine-2,4-diyl[2-(2,2,6,6-tetramethylpiperidyl)-imino]-hexamethylene-[4-(2,2,6,6-tetramethylpiperidyl)-imino] as a light stabilizer and, as the odor-suppressing stabilizer, 0.096% of a methyl ester of rosin having a Gardner-Holdt viscosity at 25°C of Y—Z3 and an acid number of 6. The peroxide prodegradant, a liquid, is first absorbed on the polypropylene flake and is added to the blender as a 2% mixture with polypropylene.

After blending, the mixture of polypropylene and additives is fed to a 38.1 mm extruder and spun through a 210 hole spinnerette at 285°C maximum melt temperature to form 621 Tex spun yarns which, in turn, were drawn to 346,5 Tex continuous filament yarns at feed and draw roll temperatures of 60 and 115°C.

Samples consisting of 10 g of drawn yarn are placed individually into each of three types of containers commonly used to package fibrous medical products: paper envelopes, on quart polyethylene bags, and 56.7 g screw-top glass jars. The yarns are sealed in their various packages and exposed to gamma-radiation at a rate of $0.21 \cdot 10^4$Gy per hour for a total of $2.5 \cdot 10^4$Gy and at a rate of 0.40 Mrads per hour for a total of $5.5 \cdot 10^4$Gy.

After two weeks of ambient storage following irradiation, the odor of the yarns in their various packages are rated by a 6-member sensory evaluation panel. The results are given in Table 1. Control A is polypropylene flake without any additives. Control B is a composition containing the same amounts of polypropylene flake, antioxidant, antacid, prodegradant and light stabilizer as Example 1, but no odor-suppressing stabilizer. The ratings for the yarn samples in the glass jars given the best indication of the efficacy of the odor-suppressing stabilizer, since the odor cannot dissipate through the jars. Evaluation time is limited to approximately 15 minutes per session. An interval of at least one hour is scheduled between successive evaluations. The values given in the first six columns of Table 1 are averages of the ratings of the six sensory evaluation panelists, based on a scale of 0—10, where 10 is odor-free. Guidelines for the selection and training of sensory panel members are given in ASTM STP 758.

### Example 2

A composition identical to the one described in Example 1, except for the use of a hydrogenated methyl ester of rosin having a Gardner-Holdt viscosity of Z—Z4 at 25°C and an acid number of 7 as the odor-suppressing stabilizer, is prepared. The composition is spun, drawn and irradiated as described in Example 1. The odor of the irradiation-sterilized fibers prepared from this composition is rated by a sensory evaluation panel as described in Example 1. The results are given in Table 1.

### TABLE 1

|  | $2.5 \cdot 10^4$Gy Total | | | $5.5 \cdot 10^4$Gy Total | | | Overall Average |
|---|---|---|---|---|---|---|---|
|  | Paper | Plastic | Glass* | Paper | Plastic | Glass* |  |
| Example 1 | 5.5 | 4.1 | 4.5 | 5.5 | 4.0 | 4.3 | 4.65 |
| Example 2 | 5.5 | 4.8 | 5.1 | 5.5 | 3.9 | 4.3 | 4.85 |
| Control A | 5.5 | 3.3 | 2.3 | 5.5 | 2.9 | 3.6 | 3.77 |
| Control B | 4.5 | 4.3 | 3.5 | 5.0 | 3.2 | 3.0 | 3.92 |

* A difference of 1.0 is considered significant.

## Claims

1. A polypropylene composition useful in preparing shaped articles that have a reduced tendency toward odor generation upon sterilization of the article by gamma-irradiation, and comprising stabilizing material in a major amount of polypropylene, wherein the polypropylene has a crystallinity of from 55% to 65%, a weight average molecular weight $M_w$ of from $3.0 \times 10^5$ to $4.0 \times 10^5$, a molecular distribution of from 5.0 to 8.0 and a melt flow of from 2.5 to 4.0 g/10 minutes at 230°C, characterized in that the composition comprises from 0.095% to 0.50% of a rosin ester as a stabilizer.

2. A composition as claimed in claim 1, further characterized in that the stabilizer is a methyl ester of rosin.

3. A composition as claimed in claim 2, further characterized in that the methyl ester of rosin is hydrogenated.

4. A method for making a shaped article that is formed from a polypropylene composition and will be sterilized by gamma-irradiation, characterized in that from 0.095 to 0.50% by weight of a rosin ester as a stabilizer is incorporated into the composition before forming the article, thereby reducing the tendency of the article to generate odor after irradiation.

5. A method for making a shaped article as claimed in claim 4, further characterized in that the rosin ester is a methyl ester of rosin.

6. A method for making a shaped article as claimed in claim 5, further characterized in that the methyl ester of rosin is hydrogenated.

**Patentansprüche**

1. Polypropylenzusammensetzung, geeignet für die Herstellung von Formkörpern mit verminderter Neigung zur Geruchsbildung nach der Sterilisierung des Gegenstandes durch Gammastrahlen, die, bezogen auf Polypropylen, stabilisierendes Material in größerer Menge enthält, wobei das Polypropylen eine Kristallinität von 55% bis 65%, ein Gewichtsmittel-Molekulargewicht $M_w$ von $3,0 \times 10^5$ bis $4,0 \times 10^5$, eine Molekularverteilung von 5,0 bis 8,0 und einen Schmelzfluß von 2,5 bis 4,0 g/10 Minuten bei 230°C aufweist, dadurch gekennzeichnet, daß die Zusammensetzung 0,095% bis 0,50% eines Kolophoniumesters als Stabilisator enthält.

2. Zusammensetzung nach Anspruch 1, weiterhin dadurch gekennzeichnet, daß der Stabilisator ein Kolophoniummethylester ist.

3. Zusammensetzung nach Anspruch 2, weiterhin dadurch gekennzeichnet, daß der Kolophoniummethylester hydriert ist.

4. Verfahren zur Herstellung eines aus einer Polypropylenzusammensetzung gebildeten Formkörpers, der durch Gamma-Bestrahlung sterilisiert wird, dadurch gekennzeichnet, daß man vor dem Ausformen des Formkörpers 0,095 bis 0,50 Gew.-% eines Kolophoniumesters als Stabilisator in die Zusammensetzung einarbeitet, wodurch die Neigung des Körpers zur Geruchsbildung nach der Bestrahlung vermindert wird.

5. Verfahren zur Herstellung eines Formkörpers nach Anspruch 4, weiterhin dadurch gekennzeichnet, daß der Kolophoniumester ein Kolophoniummethylester ist.

6. Verfahren zur Herstellung eines Formkörpers nach Anspruch 5, weiterhin dadurch gekennzeichnet, daß der Kolophoniummethylester hydriert ist.

**Revendications**

1. Composition de polypropylène utile pour préparer un article façonné ayant une tendance réduite à dégager une odeur lors de la stérilisation de l'article par irradiation gamma et comprenant une matière stabilisante dans une quantité majeure de polypropylène, dans laquelle le polypropylène a une cristallinité de 55% à 65%, un poids moléculaire moyen $M_p$ de 3,0 à $4,0 \times 10^5$, une distribution du poids moléculaire de 5,0 à 8,0 et une fluidité à l'état fondu de 2,5 à 4,0 g/10 minutes à 230°C, ladite composition étant caractérisée en ce qu'elle comprend de 0,095% à 0,50% d'un ester de colophane comme stabilisant.

2. Composition selon la revendication 1, caractérisée en ce que le stabilisant est un ester méthylique de colophane.

3. Composition selon la revendication 2, caractérisée en ce que l'ester méthylique de résine est hydrogéné.

4. Procédé pour préparer un article façonné, qui est fait d'une composition de polypropylène et qui est stérilisé par irradiation gamma, caractérisé en ce que l'on incorpore 0,095 à 0,50% en poids d'un ester de colophane comme stabilisant à la composition avant de former l'article, pour réduire la tendance de l'article à dégager une odeur après irradiation.

5. Procédé pour préparer un article façonné selon la revendication 4, caractérisé en ce que l'ester de colophane est un ester méthylique de colophane.

6. Procédé pour préparer un article façonné selon la revendication 5, caractérisé en ce que l'ester méthylique de résine est hydrogéné.